(19) Europäisches Patentamt
European Patent Office
Office européen des brevets

(11) EP 0 763 010 B1

(12) **EUROPEAN PATENT SPECIFICATION**

(45) Date of publication and mention of the grant of the patent:
**30.08.2000 Bulletin 2000/35**

(21) Application number: **95919913.4**

(22) Date of filing: **23.05.1995**

(51) Int Cl.$^7$: **C07C 209/44**, C07C 227/34

(86) International application number:
**PCT/US95/06539**

(87) International publication number:
**WO 95/32178 (30.11.1995 Gazette 1995/51)**

(54) **ENANTIOSELECTIVE PREPARATION OF OPTICALLY PURE ALBUTEROL**
ENANTIOSELEKTIVE HERSTELLUNG VON OPTISCH REINEM ALBUTEROL
PREPARATION ENANTIOSELECTIVE D'ALBUTEROL OPTIQUEMENT PURE

(84) Designated Contracting States:
**AT BE CH DE DK ES FR GB GR IE IT LI LU MC NL PT SE**

(30) Priority: **23.05.1994 US 247302**
**20.01.1995 US 376072**

(43) Date of publication of application:
**19.03.1997 Bulletin 1997/12**

(73) Proprietor: **SEPRACOR, INC.**
**Marlborough, MA 01752 (US)**

(72) Inventors:
- **GAO, Yun**
**Southborough, MA 01772 (US)**
- **ZEPP, Charles, Melvyn**
**Hardwick, MA 01037 (US)**

(74) Representative: **Ede, Eric et al**
**Fitzpatricks,**
**4 West Regent Street**
**Glasgow G2 1RS (GB)**

(56) References cited:
**WO-A-92/04314**

- **J. MED. CHEM., Volume 14, issued 17 April 1971, HARTLEY et al., "Absolute Configuration of the Optical Isomers of Salbutamol", pages 895-896.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to a method of preparing optically pure (R) and (S) albuterol. More particularly, the present invention relates to the preparation and resolution of the albuterol precursor methyl 5-[2-[(1,1-dimethylethyl) amino]-1-hydroxyethyl]-2-(phenylmethoxy) benzoate or α-[[(1,1-dimethylethyl)amino]methyl]-4-(phenylmethoxy)-1,3-benzenedimethanol with a chiral acid.

### BACKGROUND OF THE INVENTION

Albuterol, also referred to as α-[[(1,1-dimethylethyl)amino]methyl]-4-hydroxy-1,3-benzenedimethanol or as salbutamol, is a β-2 agonist useful as a bronchodilator. It possesses a high degree of selectivity between β-1 receptors (which are present in the heart) and β-2 receptors (which are present in bronchial tissue and elsewhere), for which reason it is widely used in the treatment of asthma, since in therapeutic doses it exhibits fewer cardiac side effects than many other β-agonists.

It is known that among many drugs having chiral centers one enantiomer of a racemic pair is often more active than the other in treating a medical condition. Recent data suggest that the levorotatory R-isomer of albuterol is approximately 80 times more potent than the dextrorotatory S-isomer (Hartley and Middlemis, J. Med. Chem. 14 895-896 (1971)), and preliminary research indicates that administration of the pure R-enantiomer may offer an improved therapeutic ratio.

Optical purification of a mixture of (R) and (S) methyl 5-[2-(benzyl)-t-butylamino)-1-hydroxytheyl]-2-benzyloxybenzoate using (-)ditoluoyltartaric acid is described in that literature reference. The resulting pure stereoisomer is reduced using $LiAlH_4$ to reduce the acid group to the alcohol. Further hydrogenation with removal of the benzyl group from the amino gives the acetate salt of albuterol (salbutamol).

### SUMMARY OF THE INVENTION

It is an object of the invention to provide a method for obtaining an optically pure isomer of albuterol from a phenolic precursor. It is a further object to provide a manipulatively simple synthesis of optically pure albuterol from a commercially available prochiral starting in only four steps involving one highly efficient resolution.

This and other objects, features and advantages are provided by the present invention which relates to a process for obtaining a single enantiomer of 5-[2-[(1,1-dimethylethyl)amino]-1-hydroxyethyl]-2-hydroxybenzoate, a precursor to albuterol, comprising the steps of: (a) dissolving a mixture of enantiomers of methyl 5-[2-[(1,1-dimethylethyl) amino]-1-hydroxyethyl]-2-hydroxybenzoate and a chiral acid selected from the group consisting of (-)-di-toluoyl-L-tartaric acid and (+)-di-toluoyl-D-tartaric acid in methanol by heating to form a solution; (b) allowing said solution to cool, whereby a salt of primarily one stereoisomer crystallizes; (c) separating said salt from said solution; (d) recrystallizing said salt from methanol, whereby a diastereomeric salt having greater than 90% ee of an enantiomer of methyl 5-[2-[(1,1-dimethylethyl)amino]-1-hydroxyethyl]-2-hydroxybenzoate is obtained; (e) separating said diastereomeric salt from the methanol solvent; and (f) liberating said enantiomer of methyl 5-[2-[(1,1-dimethylethyl)amino]-1-hydroxyethyl]-2-hydroxybenzoate from said diastereomeric salt by treatment with base.

In the process described above, a chiral acid such as (-)-di-toluoyl-L-tartaric acid will give the S enantiomer of methyl 5-[2-[(1,1-dimethylethyl)amino]-1-hydroxyethyl]-2-hydroxybenzoate; (+)-di-toluoyl-D-tartaric acid will give the R enantiomer of methyl 5-[2-[(1,1-dimethylethyl)amino]-1-hydroxyethyl]-2-hydroxybenzoate.

In addition, the invention encompasses a process for making optically pure albuterol from a mixture of enantiomers of 5-[2-[(1,1-dimethylethyl)amino]-1-hydroxyethyl]-2-hydroxybenzoate. The process comprises steps (a) through (f) as described above, followed by reducing the enantiomer of methyl 5-[2-[(1,1-dimethylethyl)amino]-1-hydroxyethyl]-2-hydroxybenzoate thereby forming optically active albuterol. The reduction may be accomplished with either borane-methyl sulfide or lithium aluminum hydride.

In a specific aspect the invention relates to a method for producing optically pure albuterol from methyl 5-acetylsalicylate comprising the resolution and reduction described above in combination with a method for producing 5-[2-[(1,1-dimethylethyl)amino]-1-hydroxyethyl]-2-hydroxybenzoate. According to this aspect the methyl 5-[2-[(1,1-dimethylethyl)amino]-1-hydroxyethyl]-2-hydroxybenzoate is obtained by: (a) reacting methyl 5-acetylsalicylate with hydrogen bromide in dimethyl sulfoxide, thereby forming a keto aldehyde; (b) reacting said keto aldehyde with tert-butylamine, thereby forming an α-iminoketone; and (c) reducing said α-iminoketone to provide methyl 5-[2-[(1,1-dimethylethyl)amino]-1-hydroxyethyl]-2-hydroxybenzoate.

The α-iminoketone may be reduced with either a hydride reducing agent, such as sodium borohydride, sodium cynaoborohydride, or sodium triacetoxyborohydride or by catalytic hydrogenation with a heterogeneous noble-metal catalyst, such as Pd/C, Pt/C or $PtO_2$.

It is a further object of the invention to provide a method for obtaining an optically pure isomer of albuterol

from a mono-protected albuterol precursor as well as to provide a manipulatively simple synthesis of optically pure albuterol from a commercially available prochiral starting material in only four steps involving one highly efficient resolution.

This and other objects, features and advantages are provided by the present invention which relates in one aspect to a process for obtaining a single enantiomer of albuterol, comprising:

dissolving a mixture of enantiomers of methyl 5-[2-[(1,1-dimethylethyl)amino]-1-hydroxyethyl]-2-(phenylmethoxy) benzoate and a chiral acid in methanol, ethanol or a mixture of the two by heating to form a solution, said chiral acid being selected from the group consisting of (-)-di-toluoyl-L-tartaric acid, (+)-di-toluoyl-D-tartaric acid, (-)-di-benzoyl-L-tartaric acid and (+)-di-benzoyl-D-tartaric acid;

allowing said solution to cool, whereby a salt of primarily one enantiomer crystallizes;

separating said salt from said solution;

liberating the enantiomer from said salt by treatment with a base;

reducing said enantiomer;

debenzylating said enantiomer and recovering a single enantiomer of albuterol.

In a further aspect, the invention may be characterized as a process for making optically pure albuterol, comprising:

dissolving a mixture of enantiomers of $\alpha$-[[(1,1-dimethylethyl)amino]methyl]-4-(phenylmethoxy)-1,3-benzenedimethanol and a chiral acid in methanol, ethanol or a mixture of the two by heating to form a solution, said chiral acid being selected from the group consisting of (-)-di-toluoyl-L-tartaric acid, (+)-di-toluoyl-D-tartaric acid, (-)-di-benzoyl-L-tartaric acid and (+)-dibenzoyl-D-tartaric acid;

allowing said solution to cool, whereby a salt of primarily one enantiomer crystallizes;

separating said salt from said solution;

liberating said single enantiomer from said salt by treatment with a base;

debenzylating said enantiomer and recovering optically pure albuterol.

In either process described above, a chiral acid such as (-)-di-toluoyl-L-tartaric acid or (-)-di-benzoyl-L-tartaric acid will give the S enantiomer of albuterol; (+)-di-toluoyl-D-tartaric acid or (+)-di-benzoyl-D-tartaric acid will give the R enantiomer of albuterol.

## DETAILED DESCRIPTION

The present invention relates to a more economical and efficient process for making optically pure albuterol. The method is particularly economical and efficient because it proceeds via readily available and inexpensive starting materials, as set forth in Scheme A below:

Scheme A

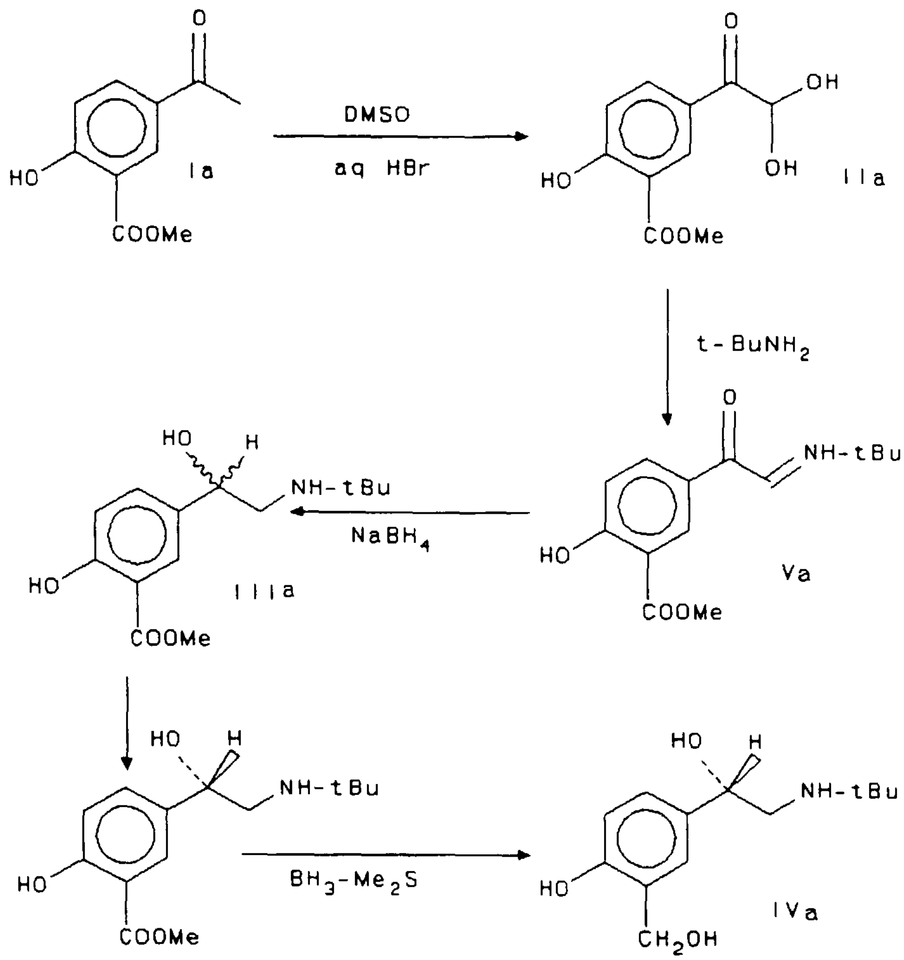


The graphic representations of racemic, ambiscalemic and scalemic or enantiomerically pure compounds used herein are taken from Mayer J. Chem. Ed. 62, 114-120 (1985). Thus, solid and broken wedges are used to denote the absolute configuration of a chiral element; wedge outlines and dotted or broken lines denote enantiomerically pure compounds of unspecified absolute configuration (e.g. structures Ib and IIIb). As usual, a wavy line indicates a mixture of enantiomers of indeterminate proportion, commonly a racemic mixture.

Many organic compounds exist in optically active forms, i.e., they have the ability to rotate the plane of plane-polarized light. In describing an optically active compound, the prefixes D and L or R and S are used to denote the absolute configuration of the molecule about its chiral center(s). The prefixes d and l or (+) and (-) are employed to designate the sign of rotation of plane-polarized light by the compound, with (-) or l meaning that the compound is levorotatory. A compound prefixed with (+) or d is dextrorotatory. There is no correlation between nomenclature for the absolute stereochemistry and for the rotation of an enantiomer. Thus, D-lactic acid is the same as (-) lactic acid, and L-lactic acid is (+). Compounds having a single chiral center exist as a pair of enantiomers which are identical except that they are non-superimposable mirror images of one another. A one-to-one mixture of enantiomers is often referred to as a racemic mixture.

The term "enantiomeric excess" is well known in the art and is defined for a resolution of ab → a+b as

$$ee_a = \left(\frac{conc.\ of\ a - conc.\ of\ b}{conc.\ of\ a + conc.\ of\ b}\right) x\ 100$$

The term "enantiomeric excess" is related to the older term "optical purity" in that both are measures of the same phenomenon. The value of ee will be a number from 0 to 100, 0 being racemic and 100 being pure, single

enantiomer. A compound which in the past might have been called 98% optically pure is now more precisely described as 96% ee. Processes that yield products of ee less than about 80% are not generally regarded as commercially attractive. Processes that yield albuterol of ee greater than about 96% are particularly attractive because the eutectic of albuterol is about 96-97% and thus substantially pure single enantiomers can be obtained by simple recrystallization of the product. "Optically pure" and "substantially optically pure" as used herein refer to albuterol of 96% ee or greater.

Arylglyoxals (IIa) are most conveniently prepared from acetophenone derivatives by the procedure of U.S. Patent 5,283,359, although other syntheses, well known to persons skilled in the art, are also suitable.

The starting material shown in Scheme A above, methyl 5-acetylsalicylate, is commercially available. Oxidation in DMSO (1.0 M) in the presence of 2 equivalents of aqueous HBr proceeds smoothly at 60°C over 20 hours to give the arylglyoxal IIa in greater than 80% yield. However, prolonged reaction times and temperatures exceeding 70°C may result in lower yields. Without further purification, this compound is treated with 1.0-1.2 eq of t-butylamine in warm toluene or ethyl acetate to give the α-iminoketone Va in greater than 70% yield. The α-iminoketone can be further purified by recrystallization from toluene/heptane and is used in the reduction after drying. The overall yield from the salicylate is greater than 60%.

The α-iminoketone Va is dissolved in a suitable solvent such as methanol and cooled with ice water. Approximately 2.5 equivalents of a hydride reducing agent are added in portions and the mixture is stirred at room temperature overnight. Thereafter the mixture is concentrated, quenched with water, and extracted into a suitable solvent, washed and recrystallized from ethyl acetate-heptane in overall yield of about 78%. The product may be analyzed for purity by any one of many methods well known in the art, an example being HPLC analysis. If the solid amino-alcohol of formula III is not greater than 95 area % pure by HPLC analysis, recrystallization is preferably repeated until this level of purity is met prior to use of the same in the resolution step.

Alternatively, the compound of formula IIIa may also be prepared directly from the corresponding α-iminoketone Va by the catalytic reductive amination with t-butylamine in the presence of heterogeneous noble-metal catalysts such as Pd/C, Pt/C, or $PtO_2$.

The precursor IIIa is resolved with a chiral acid such as (-) or (+) di-p-toluoyltartaric acid. This may be accomplished by dissolving the phenolic precursor IIIa and the chiral acid in refluxing methanol. Although this solvent may alternatively comprise ethanol or a methanol/ethanol mixture, methanol is the preferred solvent. The methanol solution is then cooled and stirred at 20-25°C for 3 to 20 hours, preferably 2 to 3 hours, thereby forming a tartrate salt in the form of a white solid. The salt is filtered off, washed with ethyl acetate to remove impurities and then dried. At this point the diastereomeric salt may represent approximately a 50% yield, of 93% ee. The solid is preferably dissolved again in refluxing methanol and the resulting solution cooled to room temperature and stored at 0° to 5°C for 10 to 20 hours. The white solid is again collected by means known in the art, such as by filtration, and dried to produce a diastereomeric salt of approximately 98.5% ee, from which the product 5-[2-[(1,1-dimethylethyl)amino]-1-hydroxyethyl]-2-hydroxybenzoate may be obtained by treatment with base, extraction, and, if desired, recrystallization from ethyl acetate.

The salicylic ester IIIa is reduced to substantially optically pure albuterol by treatment with 20 to 3 equivalents of borane-methyl sulfide complex ($BH_3•Me_2S$) in a suitable solvent, such as dichloromethane or toluene at temperatures from 50° to 60°C. It is preferred that the reaction is not heated over 60°C since higher temperatures may result in overreduction of the product. In addition, these steps are preferably performed under a dry nitrogen or argon atmosphere and the reactants and products protected from light. The reaction is quenched with methanol and worked up as usual in the art.

The highly efficient synthesis shown in Scheme A is made possible by the surprising discovery that the free phenol of formula IIIa can be resolved in good yield in a single recrystallization employing a relatively inexpensive chiral acid. Previous syntheses required either more expensive starting materials or additional protection and deprotection steps, because arriving at unresolved IIIa was considered a synthetic dead end.

In a further embodiment, optically pure albuterol may be economically and efficiently made by similarly starting with inexpensive starting materials and proceeding via a process that further minimizes the requisite steps. This alternative embodiment may be seen in reference to Scheme B as set forth below:

**Scheme B**

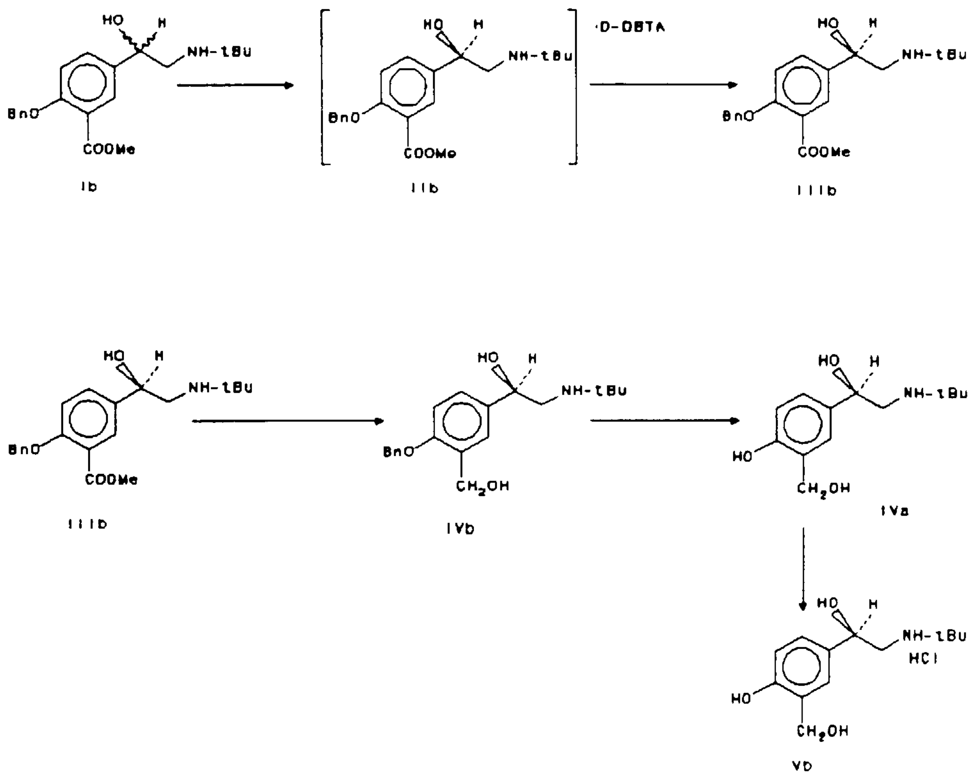


Ib IIb IIIb

IIIb IVb IVa

Vb

5-[2-[(1,1-dimethylethyl)amino]-1-hydroxyethyl]-2-(phenylmethoxy)-benzoate (structure Ib and hereinafter "compound Ib") may be prepared by procedures well known to persons skilled in the art. The starting material shown in Scheme B above, compound Ib, is commercially available from Cipla (Bombay, India).

Without further purification compound Ib is resolved with a chiral acid such as (-) or (+) di-p-toluoyltartaric acid or (-) or (+) di-benzoyltartaric acid. This may be accomplished by dissolving compound Ib and the chiral acid in refluxing methanol. The solvent may alternatively comprise ethanol or a methanol/ethanol mixture. Resolution of compound Ib may be accomplished with either about 1 mole equivalent of the tartaric acid derivative or with about 0.5 mole equivalent of the chiral acid (structure IIb and hereinafter "compound IIb salt") in the form of a solid. Compound IIb salt is filtered off, washed with ethyl acetate to remove impurities and then dried.

**Table-1**
**Resolution of racemic compound Ib**

| Scale of Compound Ib | Conditions | Yield of compound IIb | Chem. Purity | ee |
|---|---|---|---|---|
| 3 mmol | 1.0 eq of D-Ta | 31.6% | N.D. | 10.0% |
| 10 mmol | 0.5 eq of (D)-TA | 23.0% | N.D. | 10.6% |
| 200 mmol | 0.5 eq of D-DBTA | 28.7% | 99.9% area | 99.3% |
| 100 mmol | 0.5 eq of D-DBTA | 37.2% | 99.9% area | 99.0% |
| 3 mmol | 1.0 eq of D-DTTA | 37.2% | N.D. | 84.3% |

The solid, compound IIb salt, is preferably dissolved again in refluxing methanol and the resulting solution cooled to room temperature and stored at 0° to 5°C for 10 to 20 hours. The solid is again collected by means known in the art, such as by filtration, and dried to produce a diastereomeric salt of approximately 99.0% ee, from which optically active (S) or (R) methyl 5-[2-[(1,1-dimethyl ethyl)amino]-1-hydroxyethyl-2-(phenylmethoxy)-benzoate (structure IIIb and hereinafter "compound IIIb") may be obtained by treatment with base and, if desired, recrystallization from ethyl acetate.

Compound IIIb is reduced to substantially optically pure α-[[(1,1-dimethylethyl)amino]methyl]-4-(phenylmethoxy)-1,3-benzenedimethanol (structure IIc and hereinafter "4-benzyl albuterol"), by treatment with 2 to 3 equivalents of borane-THF solution ($BH_3$-THF) in a suitable solvent, such as tetrahydrofuran (THF). The solution may be refluxed and then cooled and quenched with methanol. In addition, these steps are preferably performed under anhydrous conditions, such as a dry nitrogen or argon atmosphere, and the reactants and products protected from light. The reaction is quenched with methanol and then worked up as usual in the art.

**Table-2**
**Reduction of resolved compound IIIb**

| Scale of compound IIIb | Reagent | Isolated yield of compound IVb | Chem. Purity | ee |
|---|---|---|---|---|
| 33.3 mmol | $BH_3$-THF | 73.8% | 99.8% | 99.4% |
| 33.3 mmol | $BH_3$-THF | 54.7% | 97.7% | 99.8% |

The optically pure 4-benzyl albuterol (structure IVb), may then be debenzylated to provide optically pure albuterol (structure IVa). For example, 4-benzyl albuterol may undergo debenzylation with hydrogen in the presence of a catalytic amount of Pd/C in methanol or ethanol at ambient temperature under 50 psi of hydrogen for several hours. After debenzylation the catalyst may be removed by filtration. Optically pure albuterol (structure IVa) may then be further purified and readily obtained from the filtrate as an acid salt (structure Vb) by treating the albuterol with an appropriate acid, such as anhydrous HCl, in an ethanol and ether solution.

Table-3

| Debenzylation and hydrochloride salt formation | | | |
|---|---|---|---|
| Scale of (ee%) | Yield of (R)-albuterol HCl(%)[a] (g) | Chem. purity (%) | ee (%) |
| 20.0 mmol (99.4) | 83.5 (4.60) | 99.3 | 99.6 |
| 15.0 mmol (99.8) | 80.4 (3.33) | 99.4 | 99.8 |

a. Yield after recrystallization.

The highly efficient synthesis shown in Scheme B is made possible by the surprising discovery that the mono-protected ether of compound Ib can be resolved in good yield in a single recrystallization employing a relatively inexpensive chiral acid. Previous syntheses required either more expensive starting materials or additional protection and deprotection steps.

In an alternative embodiment, optically pure albuterol may be economically and efficiently made by similarly starting with inexpensive starting materials and proceeding via a process that further minimizes the requisite steps. This alternative embodiment may be seen in reference to Scheme C as set forth below:

## Scheme C

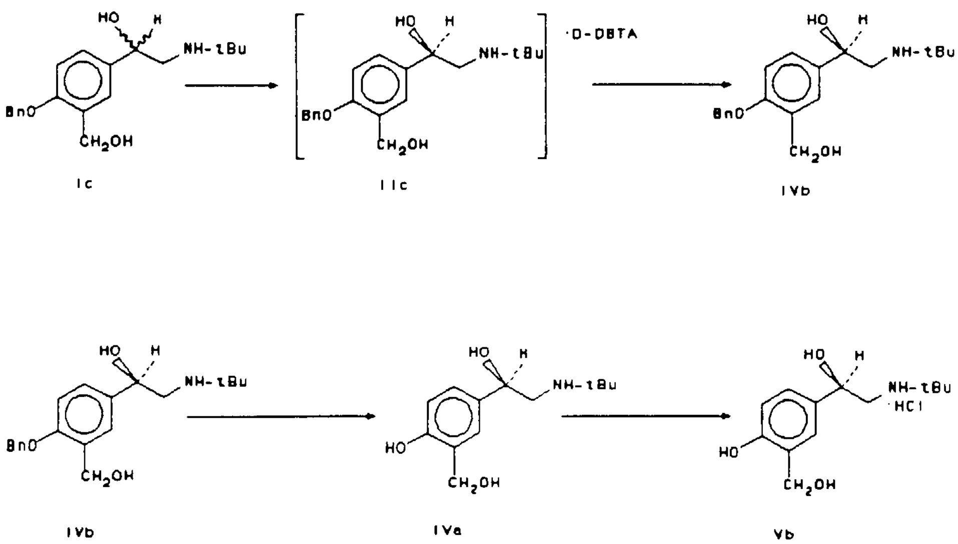


Ic IIc IVb

IVb IVa Vb

The alternative embodiment begins with a mixture of enantiomers of $\alpha$-1[[(1,1-dimethylethyl)amino]methyl]-4-(phenylmethoxy)-1,3-benzenedimethanol (structure Ic and hereinafter "4-benzyl albuterol"). As with the compound Ib above, racemic 4-benzyl albuterol (Ic) is commercially available from Cipla (Bombay, India). Alternatively, compound Ic (racemic 4-benzyl albuterol) can be prepared by reduction of racemic Ib with borane or $LiAlH_4$. Racemic 4-benzyl albuterol, as well as non one-to-one mixtures of enantiomers, may be resolved using about 1 equivalent of a chiral acid such as (-) or (+) di-p-toluoyltartaric acid (DTTA) or (-) or (+) dibenzoyltartaric acid (DBTA). The solvent may comprise ethanol or ethyl acetate, although ethanol is a preferred solvent when using dibenzoyltartaric acid as the resolving agent. The resolved chiral acid salt (structure IIc) is isolated as a solid and is treated with a base, such as 5 wt% aqueous $Na_2CO_3$ in the presence of ethyl acetate in order to obtain the resolved free base of 4-benzyl albuterol (structure IVb). The resolved free base of 4-benzyl albuterol may be further purified by crystallization from ethyl acetate and heptanes in order to achieve 99.8% chemical purity and a $\geq$ 98% ee.

Table-4

| Resolution of racemic benzyl albuterol: | | | | |
|---|---|---|---|---|
| Entry | Scale of compound Ic | Conditions | Yield[a] of compound IIc | ee |
| 1 | 30.0 mmol | 1 eq of D-DBTA ethanol[b] | 32.5% | 98.4% |
| 2 | 90.0 mmol | 1 eq of D-DBTA | 34.0% | 99.6% |
| 3 | 2 mmol | 1 eq of D-DBTA[c] | 21.7% | 94.4% |
| 4 | 2 mmol | 1 eq of D-DBTA | 50.0% | 83.5% |
| 5 | 2 mmol | 1 eq of D-DTTA ethyl acetate | 46.0% | 75.9% |

a. Yield is based upon racemic 4-benzyl albuterol compound.
b. Denatured ethanol.
c. 95% ethanol.

The free base of optically pure 4-benzyl albuterol (IVb) may then be debenzylated to form optically pure albuterol (IVa) and recrystallized in the form of an acid salt (structure Vb) as described above in reference to Scheme B.

Table-5

| Debenzylation of compound IVb and hydrochloride salt formation | | | | |
|---|---|---|---|---|
| Entry | Scale (ee%) | Yield of (R)-albuterol HCl(%)(g) | Chem. purity (%) | ee(%) |
| 1 | 9.7 mmol (98.4) | 80.9 (2.17) | 99.6 | 99.6 |
| 2 | 10.0 mmol (99.6) | 78.3(2.16) | 99.6 | 99.4 |
| 3 | 10.0 mmol (99.6) | 80.5(2.22) | 99.4 | 99.8 |

EXAMPLE 1

The synthesis of the keto aldehyde hydrate IIa was performed using a 500 mL three neck flask charged with 150mL of dimethyl sulfoxide (DMSO) and methyl 5-acetylsalicylate (I) (39g, 0.2 mol). Aq. HBr (48%, 46mL, 0.4 mol. 2.0 eq) was added dropwise over 30 min. After addition, the solution was heated at 60-70°C for ca. 20 hours (followed by TLC) until no starting material remained. The yellow mixture was poured onto 400 g of ice, stirred for 30 minutes, collected by filtration, and washed with 2x50 mL of cold water to give the keto aldehyde hydrate (IIa) (yield >80% based on dried material). The wet solid was dried at room temperature under vacuum for 4 hours and used for the next step without further purification.

The wet solid (1.0 eq, based on 0.2 mol, 100% yield) and 1.1 eq of t-butylamine (0.22 mol, 23 mL) were dissolved in 200 mL of toluene. The solution was heated at reflux for ca. 2 hours. The solution was then cooled to room temperature and washed with water (2x50 mL) and concentrated to dryness under vacuum to give crude ketoimine Va as yellow solid (33.4 g, 63% yield from methyl 5-acetylsalicylate). The crude ketoimine can be further purified for use in the $NaBH_4$ reduction. In this case, it was reduced directly with $NaBH_4$ in methanol (MeOH).

The crude ketoimine (10.6 g, 25mmol) was dissolved in 100 mL of MeOH and cooled with icewater. Solid $NaBH_4$ (2.5 g, 62.5 mmol, 2.5 eq) was added in portions with caution due to hydrogen evolution. The resulting mixture was stirred at room temperature overnight (TLC: completed) and then concentrated to dryness. The residue was quenched with 20 mL of water and extracted with 2x150 mL of ethyl acetate. The ethyl acetate solution was washed with 25 mL of $NaHCO_3$ and 25 mL of water. The solution was then concentrated to ca. 40 mL to give a slurry. The slurry was heated to dissolve the solid, and heptane (ca. 30 mL) was added. The solution was cooled to room temperature, and then stored at 3°C overnight. The solid was collected by filtration to give the first crop. The mother liquor was concentrated and recrystallized to give a second crop. Total recovery of the phenolic solid was 6.9 g (78% yield) as white solid.

EXAMPLE 2

Resolution of the phenolic precursor IIIa was performed with (-)-di-p-toluoyl-L-tartaric acid and (+)-di-p-toluoyl-D-tartaric acid, respectively.

In a representative case (examples 2.4 and 2.4a), a mixture of the phenolic precursor IIIa (1.33g, 5 mmol)

and (+)-di-p-toluoyl-D-tartaric acid (1.93 g, 5 mmol) was dissolved in refluxing MeOH (40 mL). The solution was then cooled and stirred at room temperature for 16 hours. The white solid was collected by filtration and washed with 5 mL of ethyl acetate and dried (0.86 g, 53% yield, 93% ee). The solid was then dissolved again in 18 mL of refluxing MeOH. The resulting solution was cooled to room temperature and stored at 3°C for 15 hours (overnight). The white solid was collected by filtration and dried (0.53 g, 33% yield, 98.5% ee). Results obtained analogously are summarized in the following tables:

| Example | Chiral acid | Solvent | Time at Room Temperature | Time at 3° C |
|---|---|---|---|---|
| 2.1 | (-)-L | EtOAc/ MeOH | 15 | 1.5 |
| 2.2 | (+)-D | EtOAc/ MeOH | 15 | 1.5 |
| 2.3 | (+)-D | EtOH/ MeOH | 6 | -- |
| 2.3a | -- | MeOH | 2 | 15 |
| 2.3b | -- | MeOH | 3 | 15 |
| 2.4 | (+)-D | MeOH | 16 | -- |
| 2.4a | -- | MeOH | 3 | 15 |
| 2.5 | (+)-D | MeOH | 3 | 15 |
| 2.5a | -- | MeOH | 3 | 15 |

| Example | ee% of solid | Yield % of solid | ee% of ML |
|---|---|---|---|
| 2.1 | 90(s) | 50 | 27 |
| 2.2 | 77(R) | 66 | 45 |
| 2.3 | 75(R) | 75 | -- |
| 2.3a | 95(R) | 45 | -- |
| 2.3b | 98(R) | 31 | -- |
| 2.4 | 93(R) | 53 | -- |
| 2.4a | 99(R) | 33 | -- |
| 2.5 | 90(R) | 54 | 38(S) |
| 2.5a | 99(R) | 33 | -- |

Examples identified as a or b indicate the results of an additional recrystallization of the solid obtained from the preceding crystallization. The optically pure tartrate salt can be obtained after just one more recrystallization in over 30% yield based on available isomer.

The absolute configuration of the salt from resolution with (+)-di-p-toluoyl-D-tartaric acid was correlated to R-albuterol according to the following procedure: a small amount of the salt was neutralized with saturated aqueous $NaHCO_3$ in the presence of ethyl acetate. The ethyl acetate phase was concentrated to dryness to give the enriched

free base material of formula IIIa which was then reduced with $BH_3 \cdot Me_2S$ in $CH_2Cl_2$ to give optically active albuterol. HPLC analysis in comparison with authentic (R)-albuterol confirmed that the salt has (R)-configuration at the benzyl OH group.

EXAMPLE 3

A 500 mL three-necked flask equipped with a mechanical stirrer was charged with 200 mL of DMSO (99%, ACS reagent grade) and methyl 5-acetylsalicylate (97.7%, Schweizerhall, 39.6 g, 0.2 mol, 1.0 eq). Aqueous hydrobromic acid (48%, ACS reagent grade, 34 mL, 0.3 mol, 1.5 eq) was added dropwise with stirring over 15-20 minutes. The solution was then heated at 60-65°C for 24-26 hours with stirring. Refluxing of the solution occurred at ca. 60°C. The reaction was followed by HPLC and heating was stopped when the ratio of product to starting material was greater than 95:5 by area HPLC. A μBondapak C18, 10 μm, 30 cm x 3.9 mm (Waters) column with a mobile phase consisting of 0.01 M $Na_2H_2PO_4$ - 0.002 M Octanesulfonic acid, sodium salt (pH 3.0)/Acetonitrile (75:25) was used to monitor the reaction at a UV detection wavelength of 220 nm. Prolonged reaction times and higher temperatures (over 70°C) resulted in lower yield. The solution was slowly poured onto 500 g of ice with vigorous stirring. A yellow solid precipitated out of the solution with some sticky solids being present. The mixture was stirred at 10°C for 2 hours until a fine slurry was formed during which time most of the sticky solid changed to a fine powder or small pieces. The slurry was filtered through a Buchner funnel using filter paper or DMSO-compatible filter cloth to recover the arylglyoxal product of formula II in the form of a yellow powder. The flask and solids were washed twice with 50 mL of cold water (5°C) followed by dual washes with 15 mL of cold toluene (5-10°C). The powder was retrieved and held under a vacuum for ca. 1 hour which removed most of the solvent, no additional drying was required. The powder was used without further purification having a crude yield of ca. 80% when dry with the average weight of the arylglyoxal solid falling in the range of 55-65 g.

The arylglyoxal solid (the 55-65 g., 0.2 mol) obtained from the preceding process was then transferred to a second 500 mL three-necked flas charged with 300 mL of ethyl acetate (99%, ACS reagent) thereby forming a yellow slurry. The amount of crude arylglyoxal was based on 100% yield on step one. Tertiary-butylamine (98%, Aldrich, 31.4 mL, 0.3 mol, 1.5 eq) was added to the slurry over 15-20 minutes with stirring, thereby dissolving the solids within the slurry and forming an orange solution. The mixture was heated at 40-45°C for 2-3 hours. The reaction was followed by TLC (silica gel plate pretreated with 10:1(v/v) hexane: Et3N; eluting with $CH_2Cl_2$:MeOH (20:1, v/v) containing 2 volume % of $EtN_3$), $R_f$: starting material: 0.55, ketoimine: 0.68; UV). The reaction was worked up when the starting material was almost invisible by UV detection. The solution was cooled to 20-25°C (room temperature) and separated from the dark aqueous phase. The organic phase was washed twice with 30 mL of saturated aqueous sodium chloride solution. The combined aqueous phase was not extracted and the amount of ketoimine product in the aqueous phase was ca. 5-6% of the overall yield. The organic phase was then concentrated to dryness and further dried under vacuum for 2-3 hours at room temperature yielding a yellow solid of crude ketoimine of formula Va (31-35 g, 75-82% crude yield). The crude product was used without further drying and purification in the reduction step set forth below.

A 500 mL three-necked flask was charged with the crude ketoimine from above (32 g, 0.122 mol, 1.0 eq) and 300 mL of methanol (99% ACS reagent grade) and cooled to 10-15°C. Sodium borohydride ($NaBH_4$) 98%, reagent grade) 11.6 g, 0.31 mol, 2.5 eq) was dissolved in 50 Ml of 0.2% sodium hydroxide solution and added to the ketoimine solution at 10-20°C with vigorous stirring over 30-40 minutes. This is an exothermic reaction and $H_2$ is released during addition, both of which may be controlled by using a cooling bath and by controlling the rate of addition. A slurry was formed during addition which was then stirred at 10-15°C for 20-25 minutes. The reaction usually completes after addition of the $NaBH_4$. The reaction was followed by TLC (silica gel, mobile phase:2 v/v% $Et_3N$ in 20:1 $CH_2Cl_2$/MeOH; UV, $R_f$: ketoimine, 0.44; product, 0.29) or HPLC as described hereinabove. The reaction was worked up when no arylglyoxal starting material was detected by TLC or HPLC. The slurry was concentrated below 35°C under vacuum to ca. 100-120 Ml thereby forming a dense slurry. 800 ml of ethyl acetate and 150 mL of distilled water were added to the dense slurry, stirred at 20-25°C for 30-40 minutes and allowed to settle. Although some solid slurry was present in the aqueous phase it did not affect phase separation. After separating out the aqueous phase the organic phase was washed with 50 mL of distilled water, then 50 mL of saturated NaCl solution. The organic phase was concentrated to dryness yielding a yellowish solid, weighing ca. 20-35 g greater than 95 area % pure by HPLC. The crude product was dissolved in 150-200 mL of ethyl acetate under refluxing. Thereafter, 50 mL of heptane was added to the hot solution. The weight/volume ratio of crude product to ethyl acetate is ca. 0.2:1. The volume ratio of ethyl acetate to heptane is 3:1. Although solids started to form at 40°C, the mixture was stirred and cooled to 20-25°C over 2 hours and then at 0-5°C for 4 hours. The mixture was filtered to recover the product and the flask and solids washed with 50 mL of ethyl acetate/heptane (1:1, v/v) and dried under vacuum thereby forming a white solid (33 g., 78.5% yield, 97.7 area % by HPLC). The white solid was a racemic amino-alcohol of formula IIIa. Typically the yield after recrystallization is in the range of 55-70% and more product can be recovered from the filtrate. The product was analyzed by HPLC and should be greater than 95 area % pure for use in the resolution step and, if not, then recrystallization should be repeated.

A 500 mL three-necked flask was charged with 304 mL of methanol and (+)-di-p-toluoyl-D-tartaric acid or its monohydrate (greater than 98%) (30.6 g., 76 mmol, 1.0 eq) and heated to 60-65°C with the stirring. The racemic amino-alcohol (20.0 g., 76 mmol, 1.0 eq) was added in one portion to the heated solution with stirring. The heating is necessary for the formation of the salt and the production of a homogeneous solution. The solution was then cooled to room temperature over about 1.5 hours and held at room temperature (22°C) for 4 hours, then at 0°C. The solid formed was recovered by filtration and the flask and cake rinsed with ca. 30 mL of ethyl acetate. The recovered solid was then dried under vacuum to give (21.2 g) of a white solid as the tartrate salt (80% ee in favor of the R-isomer, 42.7% yield or 85.4% yield based on available isomer). The ee was determined by HPLC on the free base, which was formed after neutralization with 5 weight % $Na_2CO_3$ in the presence of ethyl acetate. HPLC for optical purity may be conducted using a sumichiral OA 4900, 5μ, 4.6 x 250 mm column and a mobile phase of 240(hexane):140(dichloromethane):20 (methanol): 1(trifluoacetic acid) and a UV detection wavelength of 280 nm. The ee of the mother liquor was 69.3% in favor of the S-isomer. The tartrate salt was dissolved in 374 mL of methanol at reflux. The concentration of the salt in methanol was about 5.7% w/v (the range of salt concentration is 5.5-6.0% w/v in methanol). The solution was cooled to room temperature (22°C) over 1.5 hours with stirring and then stirred at room temperature for an additional 4-5 hours. The solution was further cooled to a temperature of 0°C for 1-2 hours. The solids were collected by filtration and washed with 25 mL of ethyl acetate and dried at 40°C at 28 inches of Hg for 2 hours to give the enriched tartrate salt (14.0 g, 99.1% ee of the R-isomer, 56% yield based on available isomer). The enriched salt was greater than 90% area pure by HPLC. Additional enriched salt was recovered from the filtrate by concentrating it to dryness and dissolving the residue in methanol at reflux to make a 5.0-5.5% w/v solution which was cooled at room temperature over 1.5-2 hours and stirred at room temperature for 3-4 hours at 0°C for 1 hour. The solids were recovered as above to give an enriched salt in 14% yield greater than 98% ee. The solids were combined and transferred to a flask to which 375 mL of ethyl acetate was added thereby forming a slurry. 91.2 mL of $Na_2CO_3$ solution (5 weight % aqueous solution) was added to the slurry with stirring at room temperature (22-25°C) for 30 minutes. The amount of $Na_2CO_3$ solution used represents two equivalents of $Na_2CO_3$ for each equivalent of tartrate salt. The pH of the aqueous solution is preferably ca. 9-10 (pH paper or pH meter), if the pH is less than 9, more sodium carbonate should be added to the solution to obtain the preferred pH.

The solution was then heated to ca. 30°C and the aqueous phase separated out. The organic phase was washed with 40 mL of $Na_2CO_3$ solution (5 weight %) and 28 mL of saturated NaCl solution. The solution was kept at ca. 30°C to prevent the free amino alcohol from crystallizing out from the ethyl acetate solution. The removal of the di-toluoyl-D-tartaric acid by sodium carbonate extraction was followed by the HPLC method described hereinabove. The amount of tartaric acid left should be 0% area by HPLC and if it exceeds this amount, the organic phase is preferably washed again with a sodium carbonate solution. The organic phase was dried with 10 g of anhydrous $Na_2SO_4$, and the organic solution concentrated to dryness which gave the free base as a white solid. The white solids were dissolved in 65 mL of ethyl acetate under reflux and thereafter cooled to room temperature over 1 hour with stirring. The solution remained at room temperature (22-25°C) with stirring for 1 hour and then at 0°C for 2-3 hours. The white solid formed was recovered by filtration and dried at 40°C (28 inches of Hg) for 2 hours to give an enriched R-amino-alcohol (5.0 g, 87.7% yield, greater than 99% ee, chemical purity greater than 99% area).

The R-amino-alcohol (2.67 g, 10 mmol, 1.0 eq) was added to a 100 mL flame-dried three-necked flask previously charged with 20 mL of ethylene glycol dimethyl ether (DME) anhydrous, water less than 0.005%, 99+%). This reaction and all subsequent operations were performed under dry nitrogen or argon and the reaction solutions and final products protected from light. Borane dimethyl sulfide complex ($BH_3$•$Me_2S$) (10 M, Aldrich grade) 1.6 mL, 15 mmol, 1.6 eq) was added dropwise to the above slurry over 5 minutes with stirring. Hydrogen is released and the reaction slightly exothermic (from 25°C to ca. 30°C). The solution is then heated at ca. 55°C for 3 hours. The reaction is followed by HPLC and if after 3 hours of reaction starting material is still greater than 0.3% area, it is preferred that an additional 0.1 eq of $BH_3$•$Me_2S$ be added and the solution heated at ca. 55°C for an additional hour. It is preferred that the reaction is not heated over 60°C since higher temperatures may result in overreduction of the product. Once the reaction has progressed to the desired point heating was stopped and the solution cooled to 5-10°C with use of an ice water bath. 40 mL of methanol was heated at reflux for 1 hour to destroy excess borane by forming trimethyl borane, freeing the albuterol product. 40 mL of solvent was distilled out at 60-66°C/1 atm and then an additional 20 mL of methanol was added. Another 20 mL of the solvent was distilled out at 60-66°/1 atm. The methylborate was removed azeotropically with methanol at 60-66°C. 70 mL of DME was then added followed by distilling out the solvent until the temperature reached 85°C. An additional ca. 10 mL of DME was added at 80-85°C to maintain the solvent volume at 40-45 mL during distillation. The leftover methanol was removed azeotropically with the DME. The additional DME was added slowly at reflux to prevent precipitation of solids. The final solvent volume is preferably ca. 40-45 mL. The solution was checked by HPLC: the boron-complex ($t_{R°}$ 17.0 minutes) should be less than 0.4 area on HPLC otherwise additional DME should be added and distillation continued. Then 25 mL of cyclohexane was added slowly over 10 minutes, to prevent oil-out, at ca. 80°C. Heating was then stopped and the solution cooled to ca. 20-25°C (room temperature) over 1 hour and then to 0-5°C over 1 hour and maintained at this temperature for an additional 3

hours with stirring. The solution was cooled slowly to prevent oil-out. The white powder formed was recovered by filtration. The flask and solids underwent dual washings with 10 mL of cyclohexane, the solids were dried at 50-60°C/ 28 inches of Hg for over 12 hours. This yielded a white powder, (R)-albuterol of formula IVa (weight 1.9 g, 80% yield, 98.4% area, 98% ee).

EXAMPLE-4

Racemic compound Ib (1.07g, 3 mmol) and (+)-D-di-p-toluoyltartaric acid (D-DTTA) (1.21 g, 3 mmol) are dissolved in 36 mL of methanol and refluxed for 10 min. The resulting solution is then cooled to room temperature and stirred for about 4 hours. The white solid formed is isolated by filtration and dried under vacuum to give (R)-compound IIb, the chiral acid salt of compound IIIb (0.83 g, 37.2% yield). The salt is neutralized with 5 wt% aq. $Na_2CO_3$ and extracted with ethyl acetate to give optically active (R) form of compound Ic, the free base of compound IIb, as a white solid with 84.3% ee. The optical purity (ee) is determined by chiral HPLC (Column Sumichiral OA 4900, 5μ, 4.6 x 250 mm column; Mobile phase: 240 (hexane): 140 (dichloromethane): 20 (methanol): 1 (trifluoroacetic acid) (vol); UV detection: 280 nm).

EXAMPLE-5

(+)-D-dibenzoyltartaric acid (D-DBTA) (17.9g, 50 mmol, 0.5 eq) is dissolved in 200 mL of methanol with heating to reflux. A solution of racemic compound Ib (35.7g, 100 mmol, 1.0 eq) in 200 mL of methanol is added to the above solution over 5-10 min. After addition, a white slurry is formed rapidly which is refluxed for 2 hours. The white slurry is then cooled to room temperature and stirred overnight. The solid is collected by filtration and dried under vacuum (20% ee). The solid is then re-slurried in 600 mL of methanol under reflux for 2 hours and cooled to room temperature and stirred for 4 hours. The solids are collected by filtration and dried under vacuum at room temperature for 2 hours to give a chiral acid salt, the (R) form of compound IIb (23.8 g, 94.8% ee). The salt (21.5g, 40.1 mmol) is then treated with 100 g of 5 wt% aq. $Na_2CO_3$ in 300 mL of ethyl acetate. After phase separation, the ethyl acetate phase is washed with 50 mL each of saturated aq. $NaHCO_3$ and NaCl solutions and concentrated to dryness to give the free base of compound IIb, (R) form of compound Ib, as a white solid. The crude free base is then recrystallized from 15 mL of methanol and 30 mL of ethyl acetate to give purified (R) form of compound IIIb as a white solid (12.0 g, 37.2% yield from racemic compound Ia, 99.0% ee and 99.9% purity).

EXAMPLE-6

$BH_3$-THF solution (1.0M in THF, 100 mL, 3.0 eq) is added dropwise over 30 minutes to a mixture of the (R) form of compound IIIb taken from Example-2 (12.0 g, 33.3 mmol, 1.0 eq) and 50 mL THF at room temperature under nitrogen atmosphere. The resulting solution is refluxed for 23 hours and cooled and quenched with 30 mL of methanol. The solution is concentrated to ca. 20 mL in volume and diluted with 250 mL of ethyl acetate. The solution is stirred with 40 mL of 5 wt% aq. $Na_2CO_3$ at room temperature for 30 minutes. After removal of the aqueous layer, the organic phase is washed with 40 mL each of saturated aq. $NaHCO_3$ and NaCl solution and concentrated to dryness to give a crude (R)-4-benzyl albuterol (compound IVb) as an oily foam. The crude 4-benzyl albuterol is then recrystallized from 20 mL of ethyl acetate and 20 mL of n-heptane to give pure (R)-4-benzyl albuterol as white solid (8.1 g, 73.8% yield, 99.4% ee, 99.8% purity).

EXAMPLE-7

A mixture of (R)-4-benzyl albuterol from Example-3 (6.6g, 20 mmol) and 10% Pd/C (1.32 g) in 50 mL of ethanol (denatured with 5 vol% 2-propanol) is shaken on a Parr-hydrogenator under 50 psi of hydrogen at room temperature for 2-3 hours. The catalyst is removed by filtration and the filtrate is concentrated to give crude (R)-albuterol (compound IVa). The crude albuterol (20 mmol) is dissolved in 20 mL of ethanol and treated with anhydrous HCl in ether (1.0 M, 19 mL, 0.95 eq) at 0-5°C. After 30 min at room temperature, 20 mL of methyl t-butyl ether (MTBE) is added to the mixture and the resulting mixture is stirred at room temperature for 30 min and at 0-5°C for 2 hours. The white solid (R)-albuterol hydrochloride (compound Vb) is collected by filtration and recrystallized from 52 mL of ethanol and 26 mL of MTBE to give pure (R)-albuterol hydrochloride as a white powder (4.6 g, 83.5% yield, 99.6% ee, 99.3% purity).

EXAMPLE-8

Racemic 4-benzyl albuterol (compound Ic) (0.66g, 2 mmol) and D-DTTA (0.81 g, 2 mmol) are dissolved in 5 mL of ethyl acetate with heating. The solution is then cooled to room temperature and stirred for 4 hours. The resulting

white solid is collected by filtration and dried under vacuum to give the (R) form of the chiral acid salt, compound IIc (0.66 g, 46% yield, 75.9% ee). The optical purity (ee) is determined on the free base by HPLC as in Example-4.

EXAMPLE-9

Racemic 4-benzyl albuterol (compound Ic) (0.66 g, 2 mmol) and (D-DBTA) (0.72 g, 2 mmol) are dissolved in 4 mL of ethyl acetate with heating at reflux for 10 min. The solution is then cooled to room temperature and stirred for 3 hours. The resulting solid is collected by filtration and dried to give (R) form of the chiral acid salt, compound IIc (0.07 g, 50% yield, 83.5% ee).

EXAMPLE-10

Racemic 4-benzyl albuterol (compound Ic) (0.66 g, 2 mmol) and D-DBTA (0.72 g, 2 mmol) are dissolved in 3.3 mL of 95% ethanol. The solution is heated at reflux for 10 min. and cooled to room temperature and stirred for 7 hours after seeding. The resulting solid is collected by filtration and dried to give the (R) form of the chiral acid salt, compound IIc (0.30 g, 21.7% yield, 94.4% ee).

EXAMPLE 11

D-DBTA (32.2 g, 90 mmol, 1.0 eq) is added to a hot solution of racemic 4-benzyl albuterol (compound Ic) (29.6 g, 90 mmol, 1.0 eq) in 180 mL of anhydrous denatured ethanol (type 3A, denatured with 5 vol% 2-propanol). The resulting solution is refluxed for 15 min. and cooled to room temperature over 40 min and seeded with 99% ee (R)-4-benzyl albuterol D-DBTA salt (compound IIc). The mixture is cooled to 5-10° C and stirred for 1 hour. The white solid is collected by filtration and dried at 40°C and 28 inches of Hg for 1 hour to give (R)-4-benzyl albuterol D-DBTA salt (compound IIc) (31.8 g, 50% yield, 83.6% ee). The solid is redissolved in 240 mL of ethanol at 55-60°C and the solution is cooled to room temperature and stirred at room temperature for 2 hours and at 0-5°C for 1 hour. The resulting solid is collected by filtration and dried at 40°C and 28 inches of Hg for 2 hours as (R)-4-benzyl albuterol D-DBTA salt (22.9 g, 37.1% yield, 99.3% ee). The salt (22.9 g) is then treated with 204 mL of 5 wt% aq. $Na_2CO_3$ solution in 570 mL of ethyl acetate. The solid is worked-up, and recrystallization from 30 mL of ethyl acetate and 30 mL of n-heptane gives optically pure (R)-4-benzyl albuterol free base (compound IVb) as a white powder (10.1 g, 34.1% yield from racemic Compound Ic, 99.6% ee and 99.8% purity).

EXAMPLE 12

A mixture of (R)-4-benzyl albuterol as a free base (compound IVb) from Example 8 (3.2 g, 9.73 mmol) and 10% Pd/C (0.64 g) in 24 mL of ethanol (denatured with 5 vol% 2-propanol) is shaken on a Parr-hydrogenator under 50 psi of hydrogen at room temperature for 3 hours. The catalyst is removed by filtration and the filtrate is concentrated to ca. 9 mL in volume containing crude (R)-albuterol (compound IVa) and treated with anhydrous HCl in ether (1.0 M, 9.5 mL, 0.98 eq) at 0-5°C. After 30 min. at room temperature, 9 mL of MTBE is added to the mixture and the resulting mixture is stirred at room temperature for 30 min. and at 0-5°C for 2 hours. The white solid (R)-albuterol hydrochloride is collected by filtration and recrystallized from 25 mL of ethanol and 12.5 mL of MTBE to give pure (R)-albuterol hydrochloride (compound Vb) as a white powder (2.17 g, 80.9% yield, 99.6% purity).

While the invention has been particularly shown and described with reference to preferred embodiments thereof, it will be understood by those skilled in the art that other changes in form and details may be made therein without departing from the spirit and scope of the invention.

**Claims**

1. A method for obtaining a single enantiomer of the compound methyl 5-[2-[(1,1-dimethylethyl)amino]-1-hydroxyethyl]-2-hydroxybenzoate, comprising the steps of:

   (a) dissolving a mixture of enantiomers of methyl 5-[2-[(1,1-dimethylethyl)amino]-1-hydroxyethyl]-2-hydroxybenzoate and a chiral acid, said chiral acid being selected from (-)-di-toluoyl-L-tartaric acid, and (+)-di-toluoyl-D-tartaric acid, in methanol, by heating to form a solution;

   (b) allowing said solution to cool whereby a salt of primarily one stereoisomer crystallises;

(c) separating said salt from said solution;

(d) re-crystallising said salt from methanol, whereby a diastereomeric salt having greater than 90% ee of an enantiomer of methyl 5-[2-[(1,1-dimethylethyl)amino]-1-hydroxyethyl]-2-hydroxybenzoate is obtained;

(e) separating said diastereomeric salt from the methanol solvent; and

(f) liberating said enantiomer of methyl 5-[2-[(1,1-dimethylethyl)amino]-1-hydroxyethyl]-2-hydroxybenzoate from said diasteromeric salt by treatment with a base.

**2.** A method according to claim 1, wherein the enantiomer obtained is used for making optically pure albuterol by the further steps of:
(g) reducing said liberated enantiomer of methyl 5-[2-[(1,1-dimethylethyl)amino]-1-hydroxyethyl]-2-hydroxybenzoate thereby forming optically active albuterol.

**3.** A method according to claim 2, wherein said enantiomer of methyl 5-[2-[(1,1-dimethylethyl)amino]-1-hydroxyethyl]-2-hydroxybenzoate is reduced with either borane-dimethyl sulphide, or lithium aluminium hydride.

**4.** A method according to any one of claims 1 to 3, wherein said mixture of enantiomers of methyl 5-[2-[(1,1-dimethylethyl)amino]-1-hydroxyethyl]-2-hydroxybenzoate is obtained by the steps of:

(a) reacting methyl 5-acetylsalicylate with hydrogen bromide in dimethyl sulphoxide, thereby forming a keto-aldehyde;

(b) reacting said keto-aldehyde with tert-butylamine, thereby forming an α-iminoketone; and

(c) reducing said α-iminoketone to provide methyl 5-[2-[(1,1-dimethylethyl)amino]-1-hydroxyethyl]-2-hydroxybenzoate.

**5.** A method according to claim 4, wherein said α-iminoketone is reduced with a hydride reducing agent.

**6.** A method according to claim 5, wherein said hydride reducing agent is selected from sodium borohydride, sodium cyanoborohydride, and sodium triacetoxyborohydride.

**7.** A method according to claim 4, wherein said α-iminoketone is reduced by catalytic hydrogenation.

**8.** A method according to claim 7, wherein said catalytic hydrogenation is carried out over a heterogeneous noble metal catalyst.

**9.** A method according to claim 8, wherein the noble metal catalyst is selected from Pd/C, Pt/C, and $PtO_2$.

**10.** A method for obtaining a single enantiomer useful as a precursor for albuterol, comprising:

providing a mixture of enantiomers;

selecting a chiral acid from amongst (-)-di-toluoyl-L-tartaric acid, (+)-di-toluoyl-D-tartaric acid, (-)-di-benzoyl-L-tartaric acid, and (+)-di-benzoyl-D-tartaric acid;

dissolving said mixture of enantiomers and the selected chiral acid in methanol, ethanol or a mixture of the two by heating to form a solution;

allowing said solution to cool, whereby a salt of primarily one enantiomer crystallises;

separating said salt from said solution; and

liberating the enantiomer from said salt by treatment with a base;

wherein the mixture of enantiomers is selected from either

(a) a mixture of enantiomers of methyl 5-[2-[(1,1-dimethylethyl)amino]-1-hydroxyethyl]-2-(phenylmethoxy)-benzoate; or

(b) a mixture of enantiomers of α-[[(1,1-dimethylethyl)amino]methyl]-4-(phenylmethoxy)-1,3-benzenedimethanol (racemic 4-benzyl albuterol).

**11.** A method according to claim 10, wherein the enantiomer so obtained, is de-protected by removal of the benzyl protecting group, and in the case of (a) the methyl benzoate ester is first reduced to 4-benzyl albuterol, then de-protected to recover a single enantiomer of albuterol.

**12.** A method according to claim 11, wherein the reduction is achieved using a borane complex.

**13.** A method according to claim 11 or claim 12, wherein said enantiomer is debenzylated by catalytic hydrogenation.

**14.** A method according to claim 11, further comprising, forming a slurry of said salt in methanol, ethanol or a mixture of the two, refluxing said slurry and allowing said slurry to cool whereby a salt of primarily one enantiomer crystallises.

**15.** A method according to claim 10, wherein after separating said salt from said solution, and before liberation thereof by treatment with base, the salt is recrystallised from the alcohol solvent, whereby a diastereomeric salt having greater than 90% ee of the target enantiomer is obtained; and said diastereomeric salt is separated from the alcohol solvent.

**16.** A method according to any one of claims 1, 2, 10 or 15, wherein said chiral acid is (+)-ditoluoyl-D-tartaric acid and said enantiomer is the R enantiomer.

**17.** A method according to claim 10 or claim 15, wherein said chiral acid is (+)-dibenzoyl-D-tartaric acid and said enantiomer is the R enantiomer.

**18.** A method according to claim 15 wherein said chiral acid is (+)-ditoluoyl-D-tartaric acid, said solvent is ethanol and said enantiomer is the R enantiomer.

**Patentansprüche**

**1.** Verfahren zum Abtrennen eines einzelnen Enantiomers von Methyl 5-[2-[(1,1-dimethylethyl)amino]-1-hydroxyethyl]-2-hydroxybenzoat, bei dem man:

(a) eine Enantiomerenmischung von Methyl 5-[2-[(1,1-dimethylethyl)amino]-1-hydroxyethyl]-2-hydroxybenzoat und (-)-Di-toluoyl-L-weinsäure oder (+)-Di-toluoyl-D-weinsäure als chirale Säure durch Erwärmen in Methanol löst;
(b) die Lösung abkühlen läßt, wobei ein Salz von hauptsächlich einem Stereoisomer kristallisiert;
(c) das Salz von der Lösung abtrennt;
(d) das Salz aus Methanol umkristallisiert, wobei ein diastereomeres Salz mit über 90% ee eines Enantiomers von Methyl 5-[2-[(1,1-dimethylethyl)amino]-1-hydroxyethyl]-2-hydroxybenzoat erhalten wird;
(e) das diastereomere Salz vom Lösungsmittel Methanol abtrennt; und
(f) das Enantiomer von Methyl 5-[2-[(1,1-dimethylethyl)amino]-1-hydroxyethyl]-2-hydroxybenzoat aus dem diastereomeren Salz durch Behandlung mit einer Base freisetzt.

**2.** Verfahren gemäß Anspruch 1, dadurch gekennzeichnet, daß man das erhaltene Enantiomer für die Herstellung von optisch reinem Albuterol (engl. albuterol) verwendet, indem man:
(g) das freigesetzte Enantiomer von Methyl 5-[2-[(1,1-dimethylethyl)amino]-1-hydroxyethyl]-2-hydroxybenzoat reduziert, auf diese Weise optisches aktives Albuterol bildet.

**3.** Verfahren gemäß Anspruch 2, dadurch gekennzeichnet, daß man das Enantiomer von Methyl 5-[2-[(1,1-dimethylethyl)amino]-1-hydroxyethyl]-2-hydroxybenzoat entweder mit Borwasserstoff-Dimethylsulfid oder mit Lithiumaluminiumhydrid reduziert.

**4.** Verfahren gemäß einem der Ansprüche 1 bis 3, dadurch gekennzeichnet, daß man die Enantiomerenmischung von Methyl 5-[2-[(1,1-dimethylethyl)amino]-1-hydroxyethyl]-2-hydroxybenzoat durch die Schritte erhält, daß man:

(a) Methyl 5-acetylsalicylat mit Bromwasserstoff in Dimethylsulfoxid zur Reaktion bringt, auf diese Weise ein Ketoaldehyd bildet;
(b) den Ketoaldehyd mit tert.-Butylamin zur Reaktion bringt, auf diese Weise ein $\alpha$-Iminoketon bildet; und
(c) das $\alpha$-Iminoketon reduziert, um Methyl 5-[2-[(1,1-dimethylethyl)amino]-1-hydroxyethyl]-2-hydroxybenzoat zur Verfügung zu stellen.

**5.** Verfahren gemäß Anspruch 4, dadurch gekennzeichnet, daß man das $\alpha$-Iminoketon mit einem Hydrid-Reduktionsmittel reduziert.

**6.** Verfahren gemäß Anspruch 5, dadurch gekennzeichnet, daß man das Hydrid-Reduktionsmittel aus Natriumborhydrid, Natriumcyanoborhydrid und Natriumtriacetoxyborhydrid auswählt.

**7.** Verfahren gemäß Anspruch 4, dadurch gekennzeichnet, daß man das $\alpha$-Iminoketon durch katalytische Hydrierung reduziert.

**8.** Verfahren gemäß Anspruch 7, dadurch gekennzeichnet, daß man die katalytische Hydrierung über einen heterogenen Edelmetallkatalysator ausführt.

**9.** Verfahren gemäß Anspruch 8, dadurch gekennzeichnet, daß man den Edelmetallkatalysator aus Pd/C, Pt/C und $PtO_2$ auswählt.

**10.** Verfahren zum Abtrennen eines einzelnen Enantiomers, das als Vorstufe für Albuterol einsetzbar ist, bei dem man:

eine Enantiomerenmischung zur Verfügung stellt;
eine chirale Säure aus (-)-Di-toluoyl-L-weinsäure, (+)-Di-toluoyl-D-weinsäure, (-)-Di-benzoyl-L-weinsäure und (+)-Di-benzoyl-D-weinsäure heraus auswählt;
die Enantiomerenmischung und die ausgewählte chirale Säure in Methanol, Ethanol oder ihrer Mischung durch Erwärmen löst;
die Lösung abkühlen läßt, wobei ein Salz hauptsächlich eines Enantiomers kristallisiert;
das Salz von der Lösung abtrennt; und
das Enantiomer aus dem Salz durch Behandlung mit einer Base freisetzt,

wobei die Enantiomerenmischung entweder von

(a) einer Enantiomerenmischung von Methyl 5-[2-[(1,1-dimethylethyl)amino]-1-hydroxyethyl]-2-phenylmethoxy-benzoat oder
(b) einer Enantiomerenmischung von $\alpha$-[[(1,1-Dimethylethyl)-amino]methyl-4-(phenylmethoxy)-1,3-dihydroxymethylbenzol (racemisches 4-Benzylalbuterol) ausgewählt ist.

**11.** Verfahren gemäß Anspruch 10, dadurch gekennzeichnet, daß man das so erhaltene Enantiomer durch Entfernung der Benzylschutzgruppe entschützt und im Fall (a) den Methylbenzoatester zuerst zu 4-Benzylalbuterol reduziert, dann entschützt, um ein einzelnes Enantiomer von Albuterol zu erhalten.

**12.** Verfahren gemäß Anspruch 11, dadurch gekennzeichnet, daß man die Reduktion durch Verwendung eines Borwasserstoff-Komplexes erreicht.

**13.** Verfahren gemäß Anspruch 11 oder 12, dadurch gekennzeicnet, daß man das Enantiomer durch katalytische Hydrierung debenzyliert.

**14.** Verfahren gemäß Anspruch 11, weiter aufweisend, daß man einen Schlamm des Salzes in Methanol, Ethanol oder ihrer Mischung bildet, den Schlamm unter Rückfluß kocht und den Schlamm abkühlen läßt, wobei ein Salz hauptsächlich eines Enantiomers kristallisiert.

**15.** Verfahren gemäß Anspruch 10, dadurch gekennzeichnet, daß man nach der Abtrennung des Salzes von der Lösung und vor deren Freisetzung durch Behandlung mit einer Base, das Salz aus einem alkoholischen Lösungsmittel umkristallisiert, wobei man ein diastereomeres Salz mit über 90% ee des Zielenantiomers erhält und das diastereomere Salz vom alkoholischen Lösungsmittel abtrennt.

**16.** Verfahren gemäß irgendeinem der Ansprüche 1, 2, 10 oder 15, dadurch gekennzeichnet, daß die chirale Säure (+)-Di-toluoyl-D-weinsäure ist und das Enantiomer das R Enantiomer ist.

**17.** Verfahren gemäß Anspruch 10 oder 15, dadurch gekennzeichnet, daß die chirale Säure (+)-Di-benzoyl-D-weinsäure ist und das Enantiomer das R Enantiomer ist.

**18.** Verfahren gemäß Anspruch 15, dadurch gekennzeichnet, daß die chirale Säure (+)-Di-toluoyl-D-weinsäure ist, das Lösungsmittel Ethanol ist und das Enantiomer das R Enantiomer ist.

**Revendications**

**1.** Procédé pour obtenir un énantiomère unique du composé 5-[2-[(1,1-diméthyléthyle)amino]-1-hydroxyéthyle]-2-hydroxybenzoate de méthyle, comportant les étapes consistant à :

(a) dissoudre un mélange d'énantiomères de 5-[2-[(1,1-diméthyléthyle)amino]-1-hydroxyéthyle]-2-hydroxybenzoate de méthyle et un acide chiral, ledit acide chiral étant sélectionné parmi l'acide (-)-di-toluoyle-L-tartarique et l'acide (+)-di-toluoyle-D-tartarique, dans du méthanol, en chauffant pour former une solution,
(b) permettre à ladite solution de refroidir de sorte qu'un sel constitué principalement d'un stéréo-isomère se cristallise,
(c) séparer ledit sel et ladite solution,
(d) recristalliser ledit sel à partir de méthanol, de manière à obtenir un sel diastéréo-isomère ayant un excès énantiomérique (ee) supérieur à 90 % de celui d'un énantiomère de 5-[2-[(1,1-diméthyléthyle)amino]-1-hydroxyéthyle]-2-hydroxybenzoate de méthyle,
(e) séparer ledit sel diastéréo-isomère et le solvant de méthanol, et
(f) libérer ledit énantiomère de 5-[2-[(1,1-diméthyléthyle)amino]-1-hydroxyéthyle]-2-hydroxybenzoate de méthyle dudit sel diastéréo-isomère par traitement à l'aide d'une base.

**2.** Procédé selon la revendication 1, dans lequel l'énantiomère obtenu est utilisé pour fabriquer de l'albutérol optiquement pur par les étapes supplémentaires consistant à :
(g) réduire ledit énantiomère libéré de 5-[2-[(1,1-diméthyl éthyle)amino]-1-hydroxyéthyle]-2-hydroxybenzoate de diméthyle de manière à former de l'albutérol optiquement actif.

**3.** Procédé selon la revendication 2, dans lequel ledit énantiomère de 5-[2-[(1,1-diméthyléthyle)amino]-1-hydroxyéthyle]-2-hydroxy-benzoate de méthyle est réduit à l'aide de borane-sulfure de méthyle ou d'hydrure de lithium et d'aluminium.

**4.** Procédé selon l'une quelconque des revendications 1 à 3, dans lequel ledit mélange d'énantiomères de 5-[2-[(1,1-diméthyléthyle)amino]-1-hydroxyéthyle]-2-hydroxybenzoate de méthyle est obtenu par les étapes consistant à :

(a) faire réagir du 5-acétylsalicylate de méthyle et du bromure d'hydrogène dans du sulfoxyde de diméthyle, de manière à former un céto-aldéhyde,
(b) faire réagir ledit céto-aldéhyle et de la tert-butylamine, de manière à former une α-iminocétone, et
(c) réduire ladite α-iminocétone pour fournir du 5-[2-[(1,1-diméthyléthyle)amino]-1-hydroxyéthyle]-2-hydroxy benzoate de méthyle.

**5.** Procédé selon la revendication 4, dans lequel ladite α-iminocétone est réduite à l'aide d'un agent réducteur d'hydrure.

**6.** Procédé selon la revendication 5, dans lequel ledit agent réducteur d'hydrure est sélectionné parmi du borohydrure de sodium, du cyanoborohydrure de sodium, et du triacétoxyborohydrure de sodium.

**7.** Procédé selon la revendication 4, dans lequel ladite α-iminocétone est réduite par hydrogénation catalytique.

**8.** Procédé selon la revendication 7, dans lequel ladite hydrogénation catalytique est effectuée en présence d'un catalyseur hétérogène de métaux nobles.

**9.** Procédé selon la revendication 8, dans lequel le catalyseur de métaux nobles est sélectionné parmi Pd/C, Pt/C, et $PtO_2$.

**10.** Procédé pour obtenir un énantiomère unique utile en tant que précurseur de l'albutérol, comportant les étapes consistant à :

fournir un mélange d'énantiomères,
sélectionner un acide chiral parmi l'acide (-)-di-toluoyle-L-tartarique, l'acide (+)-di-toluoyle-D-tartarique, l'acide (-)-di-benzoyle-L-tartarique et l'acide (+)-di-benzoyle-D-tartarique,
dissoudre ledit mélange d'énantiomères et l'acide chiral sélectionné dans du méthanol, de l'éthanol ou un mélange des deux en chauffant pour former une solution,
permettre à ladite solution de refroidir, de sorte qu'un seul constitué principalement d'un énantiomère se cristallise,
séparer ledit sel et ladite solution, et
libérer l'énantiomère dudit sel par traitement à l'aide d'une base,

dans lequel le mélange d'énantiomères est sélectionné parmi les mélanges suivants

(a) un mélange d'énantiomères de 5-[2-[(1,1-diméthyléthyle)-amino]-1-hydroxyéthyle]-2-(phénylméthoxy)-benzoate de méthyle, ou
(b) un mélange d'énantiomères de α-[[(1,1-diméthyléthyle)-amino]méthyle]-4-(phénylméthoxy)-1,3-benzènediméthanol (albutérol 4-benzyle racémique).

**11.** Procédé selon la revendication 10, dans lequel l'énantiomère ainsi obtenu, est déprotégé en éliminant le groupe de protection benzylique, et dans le cas du mélange (a), l'ester de benzoate méthylique est tout d'abord réduit en albutérol 4-benzyle, puis déprotégé pour récupérer un énantiomère unique d'albutérol.

**12.** Procédé selon la revendication 11, dans lequel la réaction est obtenue en utilisant un complexe de borane.

**13.** Procédé selon la revendication 11 ou 12, dans lequel on supprime le groupe benzylique dudit énantiomère par hydrogénation catalytique.

**14.** Procédé selon la revendication 11, comportant de plus les étapes consistant à former une boue dudit sel dans du méthanol, de l'éthanol ou un mélange des deux, refluer ladite boue et permettre à ladite boue de refroidir de sorte qu'un sel constitué principalement d'un énantiomère se cristallise.

**15.** Procédé selon la revendication 10, dans lequel, après séparation dudit sel et de ladite solution, et avant libération de celui-ci par un traitement à l'aide d'une base, le sel est recristallisé à partir du solvant d'alcool, de sorte qu'un sel diastéréo-isomère ayant un ee supérieur à 90 % de celui de l'énantiomère cible est obtenu, et ledit sel diastéréo-isomère est séparé du solvant d'alcool.

**16.** Procédé selon l'une quelconque des revendications 1, 2, 10 ou 15, dans lequel l'acide chiral est l'acide (+)-ditoluoyle-D-tartarique et ledit énantiomère est l'énantiomère R.

**17.** Procédé selon la revendication 10 ou 15, dans lequel ledit acide chiral est l'acide (+)-dibenzoyle-D-tartarique, et ledit énantiomère est l'énantiomère R.

**18.** Procédé selon la revendication 15, dans lequel ledit acide chiral est l'acide (+)-ditoluoyle-D-tartarique, et ledit solvant est l'éthanol et ledit énantiomère est l'énantiomère R.